# EUROPEAN PATENT SPECIFICATION

(11) **EP 4 184 514 B1**
(45) Date of publication and mention of the grant of the patent: **04.12.2024**
(21) Application number: 22153108.0
(22) Date of filing: 25.01.2022
(51) Int. Cl.: G16B 20/20, G16B 40/20, G16H 50/20

(54) **APPARATUS AND METHOD FOR DIAGNOSING CANCER USING LIQUID BIOPSY DATA**
VORRICHTUNG UND VERFAHREN ZUR KREBSDIAGNOSE UNTER VERWENDUNG VON FLÜSSIGKEITSBIOPSIEDATEN
APPAREIL ET PROCÉDÉ DE DIAGNOSTIC DU CANCER À L'AIDE DE DONNÉES DE BIOPSIE LIQUIDE

(30) Priority: 23.11.2021 KR 20210162245
(43) Date of publication of application: 24.05.2023
(73) Proprietor: Eone Reference Laboratory, Yeongsu-gu, Incheon 22014 (KR); GenesisEGO Co., Ltd., Incheon 22014 (KR)
(72) Inventor: KWON, ChangHyuk, 14051 Anyang-si, Gyeonggi-do (KR)
(74) Representative: Kurig, Thomas

(56) References cited:
- CRISTIANO STEPHEN ET AL: "Genome-wide cell-free DNA fragmentation in patients with cancer", NATURE, NATURE PUBLISHING GROUP UK, LONDON, vol. 570, no. 7761, 29 May 2019 (2019-05-29), pages 385 - 389, XP036814426, ISSN: 0028-0836, [retrieved on 20190529], DOI: 10.1038/S41586-019-1272-6

## Description

### TECHNICAL FIELD

The present disclosure relates to an apparatus and method for diagnosing cancer using liquid biopsy data.

### BACKGROUND

With advances in science and technology, people may be in a comfort zone, but they do not have access to personalized cancer treatment strategies based on their individual characteristics. Microarrays and next-generation sequencing are key tools (carter) in cancer research to quantify gene expression, DNA copy number, and microRNA activity.

Since cancer is an inherited disease, initiating an integrated test of gene mutation data and expression data is one of the best means for understanding the mechanism of carcinogenesis, predicting and preventing cancer occurrence, and finding possible treatments. In the early 90s, scientists began studying the application of artificial intelligence for information processing, data analysis, knowledge expression, and management of gene expression data. Classification using artificial intelligence is a type of data analysis that aims to provide a better understanding of cancer to enable doctors to make clinical decisions by extracting knowledge from gene expression data.

Experts may track their knowledge, but for high-dimensional gene expression data, this may be difficult and time consuming. Algorithms are available for extracting knowledge without experts, but are not suitable for good system performance. Rule-based approaches gain momentum in cancer diagnosis because making certain rules, i.e., in artificial intelligence, is a good way to express knowledge.

Decision trees are widely used to produce rules containing biologically meaningful terms, although they are sensitive enough to cause small biases in the training samples to lead to serious errors in the tree structure. Ensemble models of classification trees are as sensitive as decision trees. The rules generated by data-driven algorithms are simpler, but they fail to account for the redundant behavior of gene expressions in uncertain situations.

Although the rule-based system proposed by Komori *et al.* may be applied to predict cancer through intuitive knowledge generation from gene expression data, this approach is very vulnerable to self-learning. The hybrid fuzzy method fails to perfectly model the fuzzy system because it expresses only the rule set within the gene population by fixing the points of the membership function.

The genetic Swarm algorithm has better classification accuracy, but the if-then rules produced therefrom have more input genes and linguistic variables, making it difficult for doctors to understand. The AntBee algorithm may address the interpretability-accuracy tradeoff by producing a more readable set of rules, but the use of more complex operations with more tunable control parameters made this approach consume a lot of CPU time.

Fuzzy ontology may extract knowledge quickly, but is degraded by the scarce data distribution found in ultra-high-dimensional gene expression data. The framework within the fuzzy expert system construction transforms the crisp rules into fuzzy rules using a probabilistic global optimization procedure, but generation of the crisp rules using experts of various cancers is also difficult. In order to maintain a convincing focus on gene expression data analysis, it is better to deal with multicategory diagnostics.

In this regard, previous studies have attempted to combine classifiers from which a final decision is derived using decision by majority or fuzzy aggregation. Most ensemble classification methods are based on a black box approach whose focus is only on classification performance and provide no action for understanding the fundamental questions of medicine.

Recently, in order to utilize the significant advantage of interpretability provided by fuzzy systems, fuzzy rule-based multiclassification systems (FRBMS) using a combination method have been proposed. However, the presence of a large number of genomic variables compared to a relatively small number of patients makes the data difficult to understand. Attempts have been made to use a genetic algorithm in FRBMS to perform classifier fusion and selection together, but they did not satisfy the skewness of gene expression data.

Furthermore, underfitting should be avoided in multiple classifications where insufficient experiments result in an non-optimized robust system. In order to construct a fruitful cancer diagnosis system that pretenses multiple encounters, such as inaccurate and non-linear multicategory values, it is essential to think about ideal and suitable techniques that use the principles of rigorous data analysis. These various algorithms may achieve the most optimized results only when applied to an appropriate data set.

Whole genome sequencing data of ctDNA contains information about many molecules.

In most analyzes, only the difference in DNA mutation information or copy number variations is interpreted, and the rest of the information is mostly discarded. In most cases, attention is not paid to a change in the copy number variations of mitochondria or P/Q-arm, a comparison of total copy number, and fragment length information.

Christiano Stephen et al discloses in: "Genome-wide cell-free DNA fragmentation in patients with cancer" Nature Publishing Group UK, London, vol. 570, no. 7761, 29 May 2016, XP036814426, a method for diagnosing cancer using liquid biopsy data performed by a device, wherein an approach has been developed to evaluate fragmentation patterns of cell-free DNA across the genome, and it was found that profiles of healthy individuals reflected nucleosomal patterns of white blood cells, whereas patients with cancer had altered fragmentation profiles.

### DISCLOSURE

### TECHNICAL TASK

An aspect of the present disclosure is directed to providing an apparatus and method for diagnosing cancer, capable of determining the presence of cancer from liquid biopsy data and determining an origin and stage of cancer with an optimized method.

The aspects of the present disclosure are not limited to those mentioned above, and other aspects not mentioned herein will be clearly understood by those skilled in the art from the following description.

### TECHNICAL SOLUTION

A method and an apparatus for diagnosing cancer using liquid biopsy data is set out in the appended claims.

### BRIEF DESCRIPTION OF THE DRAWINGS

FIG. 1 is a conceptual diagram illustrating an apparatus for diagnosing cancer according to an embodiment of the present disclosure.
FIG. 2 is a flowchart illustrating a method for diagnosing cancer according to an embodiment of the present disclosure.
FIGS. 3 to 13 are conceptual diagrams illustrating the method for diagnosing cancer illustrated in FIG. 2.

### DETAILED DESCRIPTION

The advantages and features of the present disclosure and methods of achieving them will be apparent from the embodiments that will be described in detail with reference to the accompanying drawings. It should be noted, however, that the present disclosure is not limited to the following embodiments, and may be implemented in various different forms. Rather the embodiments are provided so that this disclosure will be thorough and complete and will fully convey the scope of the present disclosure to those skilled in the technical field to which the present disclosure pertains. It is to be noted that the scope of the present disclosure is defined only by the claims.

Terms used in the specification are used to describe embodiments of the present disclosure and are not intended to limit the scope of the present disclosure. In the specification, the terms in singular form may include plural forms unless otherwise specified. The expressions "comprise" and/or "comprising" used herein indicate the existence of one or more other elements other than stated elements but do not exclude presence of additional elements. Like reference denotations refer to like elements throughout the specification. As used herein, the term "and/or" includes each and all combinations of one or more of the mentioned components. It will be understood that, although the terms "first", "second", etc., may be used herein to describe various components, these components should not be limited by these terms. These terms are only used to distinguish one component from another component. Accordingly, a first component mentioned below could be termed a second component without departing from the scope of the present disclosure.

Unless otherwise defined, all terms (including technical and scientific terms) used herein have the same meaning as commonly understood by those skilled in the technical field to which the present disclosure pertains. It will be further understood that terms, such as those defined in commonly used dictionaries, should not be interpreted in an idealized or overly formal sense unless expressly so defined herein.

Hereinafter, the embodiments of the present disclosure will be described in detail with reference to the accompanying drawings.

Prior to the description, the meanings of the terms used in the present specification will be described briefly. However, it should be noted that the description of terms is used to help the understanding of the present specification, but is not to be used to limit the scope of the present disclosure in the case where the limitative details of the present disclosure are not explicitly described.

As used herein, the term "apparatus for diagnosing cancer" includes various devices capable of providing a result to a user by performing an arithmetic process.

For example, the apparatus for diagnosing cancer may include a computer, a terminal, a desktop PC, and a notebook computer as well as a smart phone, a tablet PC, a cellular phone, a Personal Communication Service (PCS) phone, a mobile terminal of a synchronous/asynchronous International Mobile Telecommunication-2000 (IMT-2000), a Palm Personal Computer (Palm PC), a personal digital assistant (PDA), etc.

In addition, the apparatus for diagnosing cancer may communicate with a server that receives a request from a client and processes information.

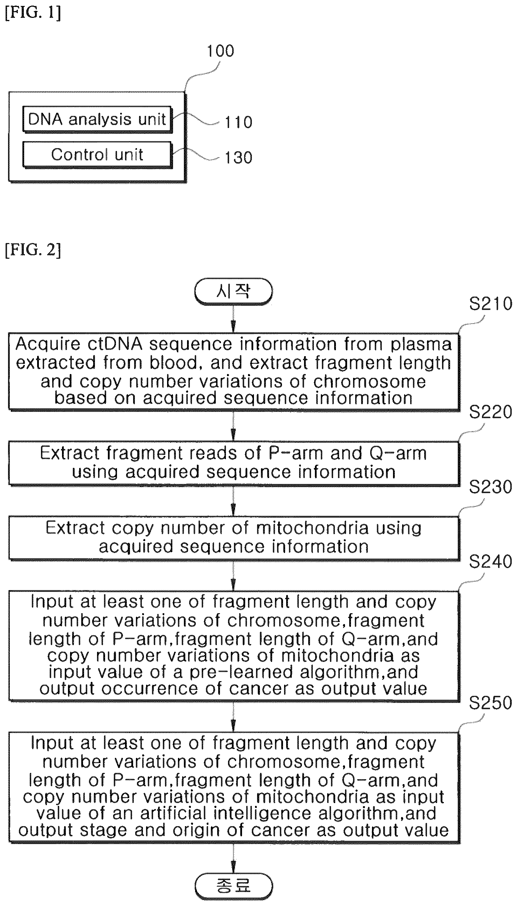

The apparatus for diagnosing cancer according to an embodiment of the present disclosure may be implemented to include at least one of the components described with reference to FIG. 1.

FIG. 1 is a conceptual diagram illustrating an apparatus for diagnosing cancer according to an embodiment of the present disclosure.

The apparatus 100 for diagnosing cancer according to an embodiment of the present disclosure may include a DNA analysis unit 110 and a control unit 130.

The DNA analysis unit 110 acquires ctDNA sequence information from plasma extracted from blood.

The DNA analysis unit 110 extracts a fragment length and a copy number variations of a chromosome based on the acquired sequence information.

The DNA analysis unit 110 extracts the fragment reads of P-arm and Q-arm using the acquired sequence information.

The DNA analysis unit 110 extracts the copy number variations of mitochondria using the acquired sequence information.

The control unit 130 may perform cancer diagnosis using the data extracted from the DNA analysis unit 110.

Specifically, the control unit 130 inputs at least one of the fragment length and the copy number variations of the sex chromosome, the fragment lengths of the P-arm, the fragment lengths of the Q-arm, and the copy number variations of the mitochondria as an input value of the pre-learned algorithm, and outputs occurrence of cancer as an output value.

In addition, the control unit 130 inputs at least one of the fragment length and the copy number variations of the sex chromosome, the fragment length of the P-arm, the fragment length of the Q-arm, and the copy number variations of the mitochondria as an input value of an artificial intelligence algorithm, and output a stage and origin of cancer as an output value.

In addition, the control unit 130 may perform monitoring to determine the recurrence and metastasis of a patient using the output value of the cancer occurrence, the stage and origin of cancer.

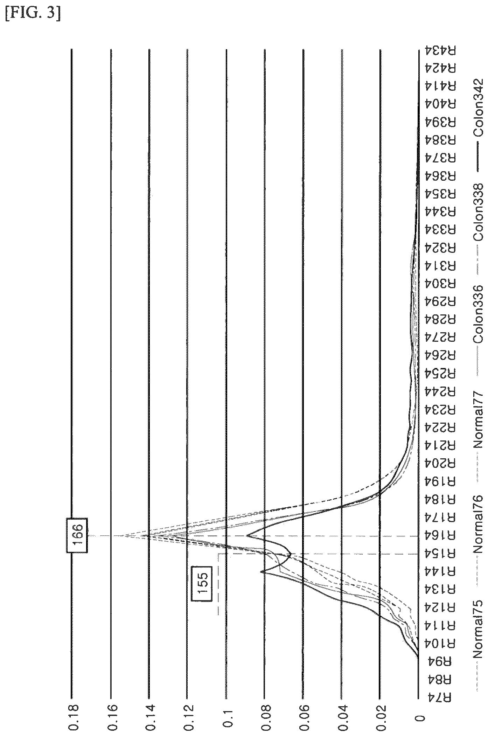

FIG. 2 is a flowchart illustrating a method for diagnosing cancer according to an embodiment of the present disclosure. FIGS. 3 to 13 are conceptual diagrams illustrating the method for diagnosing cancer illustrated in FIG. 2.

The present disclosure provides an apparatus and method for diagnosing cancer capable of discriminating the origin and stage as well as the classification of cancer or normal condition (cancer occurrence) by inputting fragment data of cell-free circulating tumor nucleic acid (ctDNA) into an artificial intelligence algorithm.

According to the present disclosure, data generated by Next Generation Sequencing (NGS) may be discriminated even at low-depth from 3X to 0.5X.

The present disclosure can significantly improve the accuracy by simultaneously inputting various data such as the difference in the copy number variations of chromosomes, the fragment length (fragment size) of the chromosomes, and the ratios of mitochondria, P-arm, and Q-arm to the pre-learned algorithm.

Accordingly, the apparatus and method for diagnosing cancer of the present disclosure can significantly reduce medical expenses by detecting cancer at an early stage, reduce cancer mortality, and can be usefully used for cancer treatment and prognosis prediction.

Referring to FIG. 2, in the apparatus for diagnosing cancer of the present disclosure, the DNA analysis unit 110 acquires ctDNA sequence information from plasma extracted from blood, and extracts the fragment length and copy number variations of chromosomes based on the acquired sequence information(S210).

The control unit 130 may control a centrifuge.

The control unit 130 may control a rpm speed of the centrifuge to maximize the amount of ctDNA (or cfDNA).

Specifically, the control unit 130 may control a centrifuge in such a way that a) in a separation method using only a one-phase one, plasma is extracted in 10 minutes at 400 g (gravity acceleration) to 1000 g, and b) in a separation method using a two-phase one, plasma is extracted at a rotational speed of 6000 g or more after applying the method a).

The plasma separated in the centrifuge may be moved to the DNA analysis unit 110 and used for analysis.

The DNA analysis unit 110 may analyze a pattern of a ctDNA fragment length in a sequenced file of whole genome sequences (WGS).

In addition, the DNA analysis unit 110 may calculate the copy number variations of ctDNA from a sequenced file of whole genome sequences (WGS).

The control unit 130 may diagnose cancer using the pattern and copy number variations of the DNA fragment length of data produced by the mass sequencing method.

The DNA analysis unit 110 may acquire sequence information (fastq format file or reads) of ctDNA from plasma, and align sequence using a human reference genome data (Human reference genome).

The DNA analysis unit 110 may align the sequence by removing sequence quality information, mapping information, and duplicate information from the sequence information.

In addition, the DNA analysis unit 110 may extract fragment lengths of an autosome, a sex chromosome and mitochondria from a first length (for example, 75) to a second length (for example, 440) using the aligned sequence information.

As illustrated in FIG. 3, the control unit 130 may secure a pattern for fragment lengths of a chromosome, and the number in a specific fragment length (for example, 155 or 166) is shown to be different from a normal case and a cancer case.

In addition, the DNA analysis unit 110 extracts a fragment read of P-arm and a fragment read of Q-arm using the acquired sequence information (S220).

The DNA analysis unit 110 extracts the fragment lengths of the P-arm and the Q-arm based on a centromere of a chromosome (all chromosomes).

In addition, the DNA analysis unit 110 may extract sequence information having a GC content and a mapping rate equal to or greater than a reference value (cut-off) using the aligned sequence information.

The DNA analysis unit 110 may divide each region into bin regions having a size equal to or greater than a specific size and normalize an amount of each section.

The DNA analysis unit 110 may calculate a Z-score using a mean and standard deviation for each bin region, and extract the copy number variations by quantifying the Z-score.

Specifically, the DNA analysis unit 110 of the present disclosure may extract a read (sequence information) having a GC content and a mapping rate (mappability) equal to or greater than a reference value using the aligned sequence information (data).

In addition, the DNA analysis unit 110 may divide each chromosomal region into bins of 5 kb or more (5 KB to 10 MB) to check and normalize an amount of each section.

The DNA analysis unit 110 may calculate the Z-score using the mean and standard deviation obtained based on the bin.

The DNA analysis unit 110 may quantify the value of the Z-score to digitize data on a difference in the copy number.

Specifically, the DNA analysis unit 110 may calculate a difference between copy number variations (CNV) of autosomes, sex chromosomes, and mitochondria using sequence alignment data.

The DNA analysis unit 110 may remove ambiguous reads (sequence information) from the sequence alignment data, align only perfectly matched reads, and extract a read having a GC content and a mapping rate (Mappability) equal to or greater than a reference value from the aligned sequences.

The DNA analysis unit 110 may divide each region into bins of 5 kb or more (5 KB to 10 MB) to calculate a Z-score.

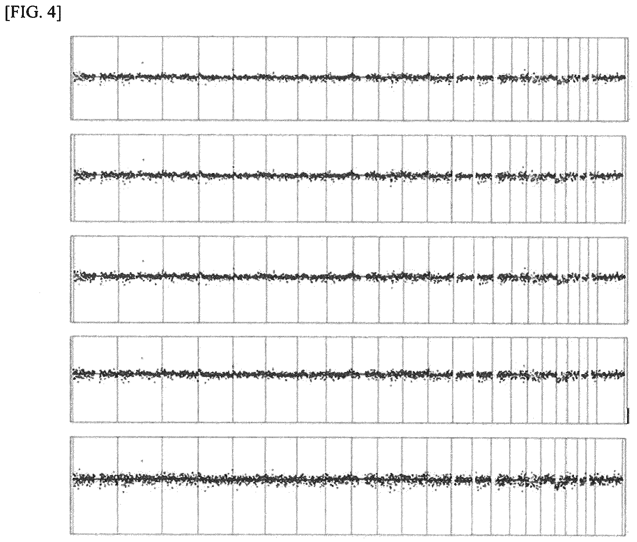

FIG. 4 illustrates the results at various read depths from 0.1X to 5X.

FIG. 4 is a graph showing the results of the copy number variations according to the read depth, and graphs of 5X, 3X, 1X, 0.5X, and 0.1X are shown in plan view.

Referring to FIG. 4, as the final result graph, a depth of up to 0.5X is appropriate, but from a depth of 0.1X, the score rapidly changes and falls, and it is determined that there is a change in the copy number variations due to the low depth of the result of a normal person without a copy number variations change.

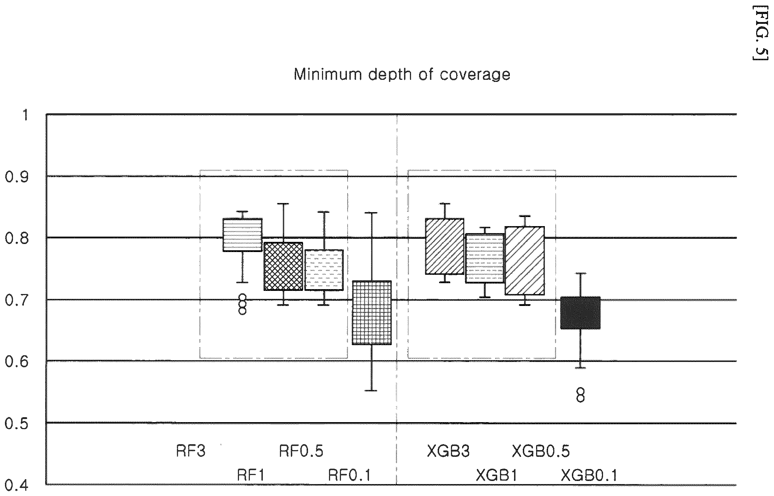

As in the random forest result of FIG. 5, the results of 0.5X to 5X are almost constant, but the value of 0.1X has a great influence on the overall result.

The DNA analysis unit 110 extracts the copy number variations of mitochondria using the acquired sequence information (S230).

Specifically, the DNA analysis unit 110 calculates a sequence information depth of mitochondria, and calculates an average sequence information depth of an autosome.

The DNA analysis unit 110 extracts a copy number variations of mitochondria by dividing the sequence information depth of mitochondria into the average sequence information depth of the autosome.

The DNA analysis unit 110 may calculates the copy number variations of mitochondria as follows.

Calculation of the copy number variations of mitochondria = mitochondrial read depth/average read depth of autosomes.

Thereafter, the control unit 130 may input at least one of the fragment length and the copy number variations of the chromosome, the fragment length of the P-arm, the fragment length of the Q-arm, and the copy number variations of the mitochondria as an input value of the pre-learned algorithm, and output occurrence of cancer as an output value (S240).

The pre-learned algorithm may include at least one algorithm of Random Forest (RF), Support Vector Machine (SVM), eXtra Gradient Boost (XGB), Decision Tree Classifier (DTC), K-nearest Neighbors (KNN), Gaussian Naive Bayes (GNB), Stochastic Gradient Descent (SGD), Linear Discriminant Analysis (LDA), Ridge Regression, Lasso, and Elastic net.

In addition, the control unit 130 inputs at least one of the fragment length and the copy number variations of the chromosome, the fragment length of the P-arm, the fragment length of the Q-arm, and the copy number variations of the mitochondria as well as occurrence of cancer as an input value of an artificial intelligence algorithm, and outputs a stage and origin of cancer as an output value (S250).

The control unit 130 may integrate a fragment length of the chromosome and ratio data of the P-arm and Q-arm, and integrate the copy number variations of the autosome and ratio data of the mitochondria.

Thereafter, the control unit 130 may input the integrated ratio data as input values.

The artificial intelligence algorithm may include Random Forest (RF), Support Vector Machine (SVM), eXtra Gradient Boost (XGB), Decision Tree Classifier (DTC), K-nearest Neighbors (KNN), Gaussian Naive Bayes (GNB), Stochastic Gradient Descent (SGD), Linear Discriminant Analysis (LDA), and algorithms such as Ridge Regression, Lasso, and Elastic net classifiers.

The artificial intelligence algorithm may mean algorithm designed and learned to extract a stage and origin of cancer as well as occurrence of cancer, and may be pre-stored by the control unit 130 and periodically updated.

In addition, the control unit 130 may use the integrated data to inflate a sample by tens to hundreds of times using various sample expansion methods such as a Generative Adversarial Network (GAN), SMOTE, and Denoising Autoencoder, and discriminate the presence of cancer with a classifier of artificial intelligence and deep learning.

In addition, the control unit 130 may use the generated integrated data to inflate a sample by tens to hundreds of times using various sample expansion methods such as a Generative Adversarial Network (GAN), SMOTE, and Denoising Autoencoder, and discriminate the stage and origin of cancer with a classifier of artificial intelligence and deep learning.

Through this configuration, the present disclosure can introduce an artificial intelligence learning approach to a biological analyte that may affect cancer, and interpret cancer and predict the origin and stage of cancer to detect cancer at an early stage and reduce the mortality rate. Moreover, the present invention can also provide a useful method for significantly reducing medical expenses by performing cancer treatment and prognosis prediction.

In addition, the present disclosure can contribute to the happiness and welfare of mankind by providing a possible service for the entire nation at a low price with a method and apparatus for predicting cancer even at a depth as low as 0.5X.

Hereinafter, the experimental results according to the present disclosure will be described with reference to the accompanying drawings.

The accuracy of cancer diagnosis is as follows.

As for the fragment length, unlike those previously reported, the results using the total length that may be inferred from the fragment length in comparison between 155 bp or less and the whole showed slightly better results at the median value.

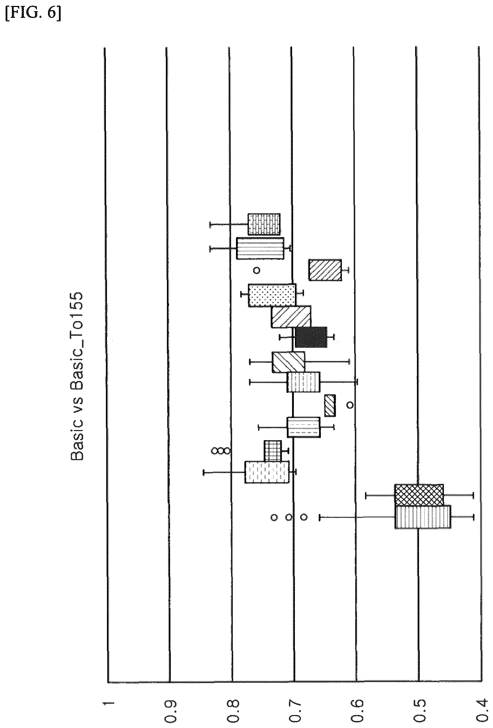

Referring to FIG. 6, FIG. 6 is a comparison diagram of a graph using Basic using the entire region and using only up to 155 bp.

In this experiment, the results of each algorithm were compared, in the order of Stochastic Gradient Descent (SGD), Random Forest (RF), Support Vector Machines (SVM), Decision Tree Classifier (DTC), K-nearest Neighbors (KNN), Gaussian Naive Bayes (GNB), and eXtra Gradient Boost (XGB ).

The left one is Basic and the right one is the result up to 155 bp. All results of Basic are the result of adding copy number variations (CNV), and it is an accuracy value.

The result of capturing only 155 bp in a difference in the pattern of FIG. 3 or the result of 155 bp or less (74 bp to 155 bp) + 180 bp or more (180 bp to 220 bp) was not higher than the result of using the entire 75 bp to 440 bp.

This experiment identified that the result having all regions (75bp ~ 440bp) was more beneficial.

### 2) CNV, Fragmentation, P/Q-arm regions and analysis

The data of the copy number variation (CNV) was segmented into a 5 kb region and the result data of a z-score was used, and the basic analysis methods of the author's previous NIPT patents 1018177850000 and 1018171800000 were used.

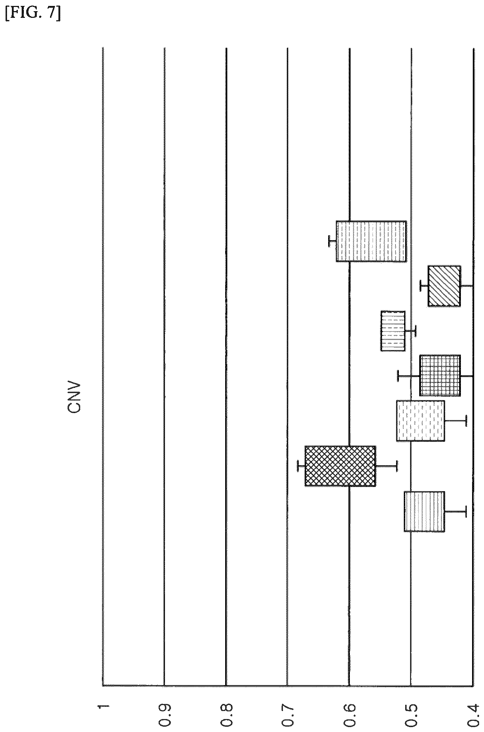

As illustrated in FIG. 7, the results of 1kb to 5kb, 1mb, etc. were almost similar, so they are shown only in the case of segmentation into 5kb region, and the result of Random Forest had a median value of 0.61.

For the fragmentation region, numerical values using the results of 1) above and the entire region of 75 bp to 440 bp in FIG. 3 were used.

In the normalization process of fragmentation data, the exact fragment length was determined using the paired-end length of 1275 samples, and the number of distributions for each size was determined.

All data may be expressed as a ratio of each length with respect to the total fragment or obtained using a Z-score.

Each region of the P/Q-arm followed the general criteria for classifying the centromere into the P-arm region on the upper side and the Q-arm region on the lower side as shown in Table 1 below, and followed the criteria for classification and distribution in general in the UCSC Genome Browser.

**[Table 1]**

| Regions of P-arm and Q-arm selected based on a centromere | | | | | | |
|---|---|---|---|---|---|---|
| | chr1 | chr2 | chr3 | chr4 | chr5 | chr6 |
| P-arm | 125000000 | 93300000 | 91000000 | 50400000 | 48400000 | 61000000 |
| Q-arm | 249250621 | 243199373 | 198022430 | 191154276 | 180915260 | 171115067 |

| | chr7 | chr8 | chr9 | chr10 | chr11 | chr12 |
|---|---|---|---|---|---|---|
| P-arm | 59900000 | 45600000 | 49000000 | 40200000 | 53700000 | 35800000 |
| Q-arm | 159138663 | 146364022 | 141213431 | 135534747 | 135006516 | 133851895 |

| | chr13 | chr14 | chr15 | chr16 | chr17 | chr18 |
|---|---|---|---|---|---|---|
| P-arm | 17900000 | 17600000 | 19000000 | 36600000 | 24000000 | 17200000 |
| Q-arm | 115169878 | 107349540 | 102531392 | 90354753 | 81195210 | 78077248 |

| | chr19 | chr20 | chr21 | chr22 | chr23 | chr24 |
|---|---|---|---|---|---|---|
| P-arm | 26500000 | 27500000 | 13200000 | 14700000 | 60600000 | 12500000 |
| Q-arm | 59128983 | 63025520 | 48129895 | 51304566 | 155270560 | 59373566 |

The distribution of the length of the paired-end of the P-arm was calculated, and only a region of 100 bp to 400 bp was used, and the ratio of each region and P/Q-arm was used for analysis.

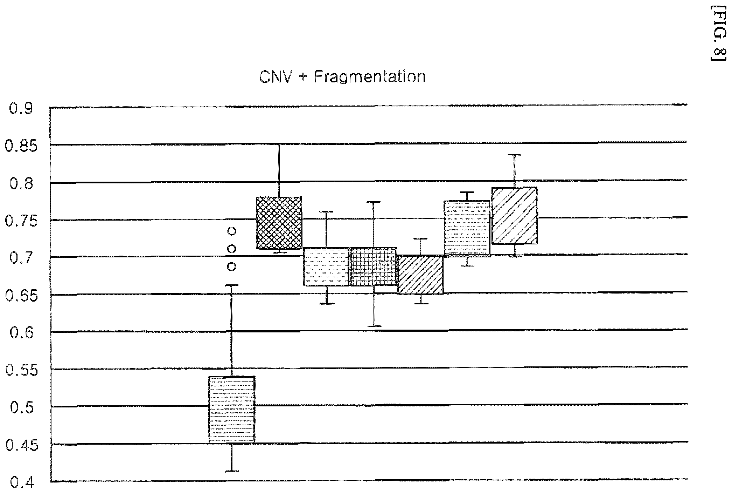

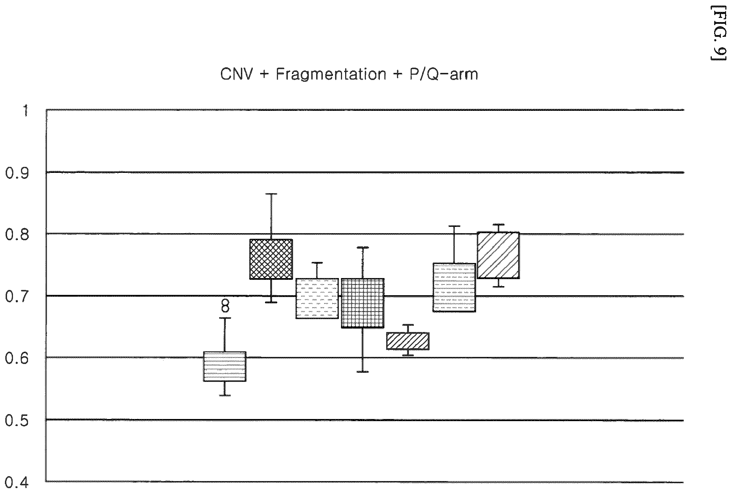

In FIG. 9, a better value than the existing Basic (CNV + Fragmentation) result (FIG. 8) was obtained from the data (CNV + Fragmentation + P/Q-arm) result using the ratio of P/Q-arm, and the median value of 0.78, which was improved by about 0.2 compared to the conventional one, was obtained in the XGB result. In addition, it was identified that the median value was improved as a whole.

### 3) Analysis and results of adding mitochondria to Basic

In addition to the data of Basic (CNV + Fragmentation), in order to reflect the copy number variations of mitochondria showing significant results in cancer, it was calculated using (average read depth of mitochondria)/(average read depth of autosomes) in order to reflect the number of mitochondria compared to the total.

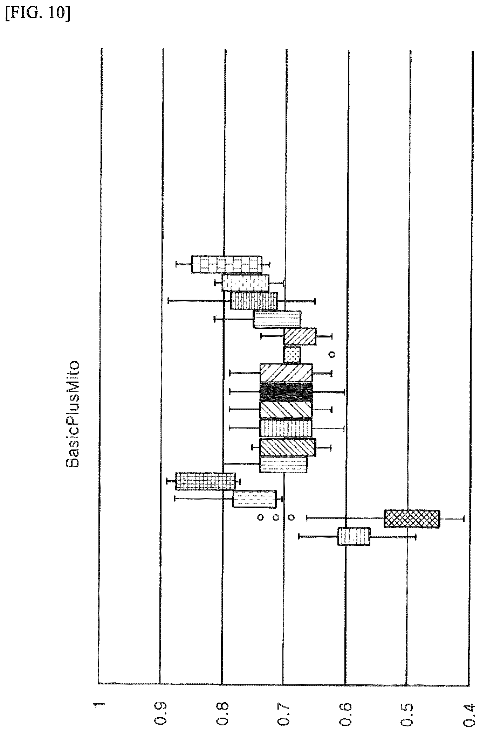

In the result of FIG. 10, there was an improvement in accuracy of 3 to 5%, unlike the existing data. The result of RF showed a median value of 80%, and there were also results with a maximum accuracy of 83%. Although XGB had similar results, RF showed good results with less distribution of median values and variances as a whole.

FIG. 10 illustrates Stochastic Gradient Descent (SGD), Random Forest (RF), Support Vector Machines (SVM), Decision Tree Classifier (DTC), K-nearest Neighbors (KNN), Gaussian Naive Bayes (GNB), and eXtra Gradient Boost (XGB) in order, where the results of Basic and BasicPlusMito (adding mitochondria to Basic) are drawn in pairs in order, and shows a result of performing the result of 10 fold 10 times.

**[Table 2]**

| Detection approach | Patients analyzed | Patients detected | Fraction of patients detected | 95% CI |
|---|---|---|---|---|
| SmartCfDNA | 126 | 106 | 84% | 78%-89% |
| DELFI | 126 | 103 | 82% | 74%-88% |
| Mutations | 126 | 83 | 66% | 57%-74% |

In 98% of specificities, DELFI cited values in its entirety, and SmartCfDNA is the result of RF.

### 4) Ability to determine the origin of each cancer

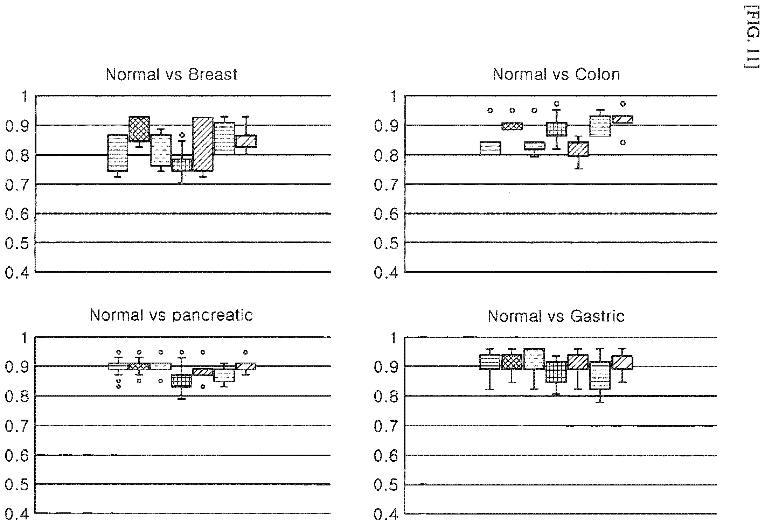

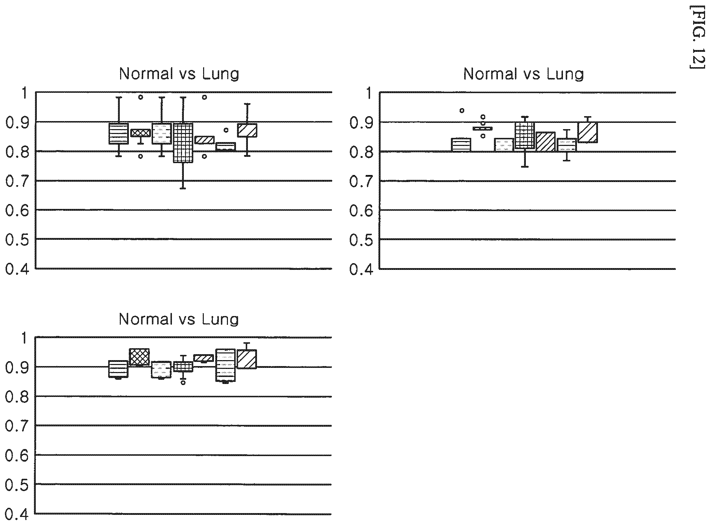

In the present disclosure, the most important thing after distinguishing between a normal state and cancer is to determine what kind of cancer it is. Referring to FIGS. 11 and 12, it is possible to determine breast cancer, colon cancer, pancreatic cancer, stomach cancer, lung cancer, ovarian cancer, and biliary tract cancer using modules learned from each dataset.

When using RF, the accuracy in detecting lung cancer is 85%, but there is a distinguishing power of 86%, 90%, 89%, 92%, 86%, 89%, and 93%. The origin of cancer may be determined, while most lung cancers are detected with accuracy of 90% or more.

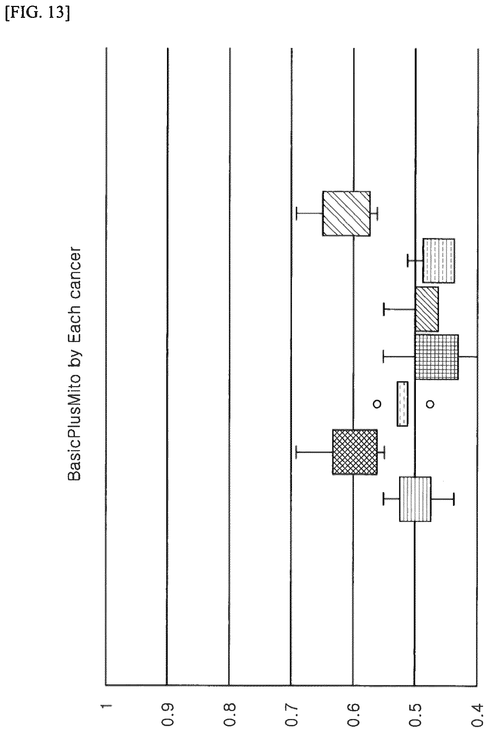

Referring to FIG. 13, the ability to distinguish 7 cancers of the present disclosure showed 60% in RF (55-65% in 95% CI), and 63% in XGB (57-66% in 95% CI), and the ability to predict the top two cancers showed 78% both in RF and XGB.

The operations and functions of the apparatus for diagnosing cancer described above may be inferred and applied in the same/similar way to the method for diagnosing cancer.

According to the present disclosure as described above, the present disclosure may provide a method for diagnosing cancer capable of diagnosing the presence of cancer and discriminating the origin and stage of cancer with an optimized method.

In addition, the present disclosure can provide a method for diagnosing cancer with higher accuracy compared to the related art.

The method according to an embodiment of the present disclosure described above may be implemented as a program (or an application) to be executed in combination with a server, which is hardware, and stored in a medium.

The above-described program may include a code encoded by a computer language such as C, C++, JAVA, or a machine language, which a processor (CPU) of the computer can read through a device interface of the computer, such that the computer reads the program and performs the methods implemented with the program. The code may include functional codes associated with the function that defines functions necessary to perform the methods, and may include a control code associated with an execution procedure necessary for the processor of the computer to perform the functions in a predetermined procedure. Furthermore, the code may further include additional data necessary for the processor of the computer to perform the functions or a memory reference-related code associated with the location (address) of the internal or external memory of the computer, at which the media needs to be referred. In addition, when the processor of the computer needs to communicate with any other remote computer or any other remote server to perform the functions, the code may further include a communication-related code associated with how to communicate with any other remote computer or server using the communication module of the computer, and what data or media should be transmitted or received during communication.

The storing media may mean the media that does not store data for a short period of time such as a register, a cache, a memory, or the like but semi-permanently stores to be read by the device. Specifically, for example, the storing media include, but are not limited to, ROM, RAM, CD-ROM, magnetic tape, floppy disk, optical data storage device, and the like. That is, the program may be stored in various recording media on various servers that the computer can access, or various recording media on the computer of the user. In addition, the media may be distributed to a computer system connected to a network, and a computer-readable code may be stored in a distribution manner.

The operations of a method or algorithm described in connection with the embodiments of the present disclosure may be embodied directly in hardware, in a software module executed by hardware, or in a combination thereof. The software module may reside on a Random Access Memory (RAM), a Read Only Memory (ROM), an Erasable Programmable ROM (EPROM), an Electrically Erasable Programmable ROM (EEPROM), a Flash memory, a hard disk, a removable disk, a CD-ROM, or a computer readable recording medium in any form well known in the technical field to which the present disclosure pertains.

Although the embodiments of the present disclosure have been described with reference to the attached drawings, those skilled in the technical field to which the present disclosure pertains will understand that the present disclosure may be practiced in other detailed forms without departing from the scope of the present disclosure. Therefore, it should be understood that the above-described embodiments are exemplary in all aspects rather than being restrictive.

## Claims

1. A method for diagnosing cancer using liquid biopsy data performed by a device, the method comprising:
a) acquiring ctDNA sequence information from plasma extracted from blood, and extracting fragment lengths and copy number variations of a sex chromosome, an autosome and mitochondria based on the acquired sequence information; and
b) extracting fragment reads and fragment lengths of the P-arm and Q-arm based on a centromere of the sex chromosome
c) calculate a sequence read depth of the mitochondria, and calculate an average sequence read depth of the autosome and extract the copy number variations of the mitochondria by dividing the sequence information depth of the mitochondria by the average sequence information depth of the autosome; and
d) inputting at least one of the fragment lengths and the copy number variations of the sex chromosome, the fragment lengths of the P-arm, the fragment lengths of the Q-arm, and the copy number variations of the mitochondria as an input value of a pre-learned algorithm, and outputting occurrence of cancer as an output value; and
e) inputting at least one of the fragment length and the copy number variations of the sex chromosome, the fragment lengths of the P-arm, the fragment lengths of the Q-arm, and the copy number variations of the mitochondria as an input value of an artificial intelligence algorithm, and outputting a stage and origin of cancer as an output value.

2. The method of claim 1, wherein operation a) includes: aligning the sequence information using human reference genome data, and extracting the fragment lengths of the autosome and the sex chromosome from a first length to a second length using the aligned sequence information.

3. The method of claim 1 wherein in operation d), the pre-learned algorithm, and in operation e), the artificial intelligence algorithm includes at least one algorithm of Random Forest (RF), Support Vector Machine (SVM), eXtra Gradient Boost (XGB), Decision Tree Classifier (DTC), K-nearest Neighbors (KNN), Gaussian Naive Bayes (GNB), Stochastic Gradient Descent (SGD), Linear Discriminant Analysis (LDA), Ridge Regression, Lasso, and Elastic net.

4. An apparatus for diagnosing cancer using liquid biopsy data, the apparatus including:
a DNA analysis unit for acquiring ctDNA sequence information from plasma extracted from blood, extractingafragment lengths
and copy number variations of a sex chromosome, an autosome and mitochondria based on the acquired sequence information, extracting fragment reads and fragment lengths of the P-arm and Q-arm based on a centromere of the sex chromosome, calculating a sequence read depth of the mitochondria, and calculating an average sequence read depth of the autosome and extracting the copy number variations of the mitochondria by dividing the sequence information depth of the mitochondria by the average sequence information depth of the autosome; and
a control unit for inputting at least one of the fragment lengths and the copy number variations of the sex chromosome, the fragment lengths of the P-arm, the fragment lengths of the Q-arm, and the copy number variations of the mitochondria as an input value of a pre-learned algorithm, and outputting occurrence of cancer as an output value,
wherein the control unit is configured to input at least one of the fragment length and the copy number variations of the sex chromosome, the fragment lengths of the P-arm, the fragment lengths of the Q-arm, and the copy number variations of the mitochondria as an input value of an artificial intelligence algorithm, and output a stage and origin of cancer as an output value.

5. The apparatus of claim 4, wherein the DNA analysis unit is further configured to:
aligning the sequence information using human reference genome data; and
extract the fragment lengths of the autosome and the sex chromosome from a first length to a second length using the aligned sequence information.

6. The apparatus of claim 4, wherein the pre-learned algorithm and the artificial intelligence algorithm each include at least one algorithm of Random Forest (RF), Support Vector Machine (SVM), eXtra Gradient Boost (XGB), Decision Tree Classifier (DTC), K-nearest Neighbors (KNN), Gaussian Naive Bayes (GNB), Stochastic Gradient Descent (SGD), Linear Discriminant Analysis (LDA), Ridge Regression, Lasso, and Elastic net.

## Patentansprüche

1. Verfahren zur Diagnose von Krebs unter Verwendung von Flüssigbiopsiedaten, durchgeführt von einer Vorrichtung, wobei das Verfahren umfasst:
a) Erfassen von ctDNA-Sequenzinformationen aus Plasma, extrahiert aus Blut, und Extrahieren von Fragmentlängen und Kopienzahlvarianten eines Geschlechtschromosoms, eines Autosoms und von Mitochondrien auf der Grundlage der erfassten Sequenzinformationen; und
b) Extrahieren von Fragment-Reads und Fragmentlängen des P-Arms und Q-Arms auf der Grundlage eines Zentromers des Geschlechtschromosoms;
c) Berechnen einer Sequenz-Read-Tiefe der Mitochondrien und Berechnen einer durchschnittlichen Sequenz-Read-Tiefe des Autosoms und Extrahieren der Kopienzahlvarianten der Mitochondrien durch Dividieren der Sequenzinformationstiefe der Mitochondrien durch die durchschnittliche Sequenzinformationstiefe des Autosoms; und
d) Eingeben von mindestens einem der Fragmentlängen und der Kopienzahlvarianten des Geschlechtschromosoms, der Fragmentlängen des P-Arms, der Fragmentlängen des Q-Arms und der Kopienzahlvarianten der Mitochondrien als einen Eingabewert eines vorab erlernten Algorithmus, und Ausgeben des Auftretens von Krebs als einen Ausgabewert; und
e) Eingeben von mindestens einem der Fragmentlängen und der Kopienzahlvarianten des Geschlechtschromosoms, der Fragmentlängen des P-Arms, der Fragmentlängen des Q-Arms und der Kopienzahlvarianten der Mitochondrien als einen Eingabewert eines künstliche Intelligenz-Algorithmus und Ausgeben eines Stadiums und Ursprungs von Krebs als einen Ausgabewert.

2. Verfahren nach Anspruch 1, wobei Vorgang a) einschließt:
Ausrichten der Sequenzinformationen unter Verwendung von menschlichen Referenzgenomdaten, und
Extrahieren der Fragmentlängen des Autosoms und des Geschlechtschromosoms von einer ersten Länge zu einer zweiten Länge unter Verwendung der ausgerichteten Sequenzinformationen.

3. Verfahren nach Anspruch 1, wobei in Vorgang d) der vorab erlernte Algorithmus und in Vorgang e) der künstliche Intelligenz-Algorithmus mindestens einen Algorithmus von Random Forest (RF), Support Vector Machine (SVM), eXtra Gradient Boost (XGB), Decision Tree Classifier (DTC), K-nearest Neighbors (KNN), Gaussian Naive Bayes (GNB), Stochastic Gradient Descent (SGD), Linear Discriminant Analysis (LDA), Ridge Regression, Lasso und Elastic net einschließt.

4. Vorrichtung zur Diagnose von Krebs unter Verwendung von Flüssigbiopsiedaten, wobei die Vorrichtung einschließt:
eine DNA-Analyseeinheit zum Erfassen von ctDNA-Sequenzinformationen aus Plasma, extrahiert aus Blut, zum Extrahieren von Fragmentlängen und Kopienzahlvarianten eines Geschlechtschromosoms, eines Autosoms und von Mitochondrien auf der Grundlage der erfassten Sequenzinformationen, zum Extrahieren von Fragment-Reads und Fragmentlängen des P-Arms und Q-Arms auf der Grundlage eines Zentromers des Geschlechtschromosoms, zum Berechnen einer Sequenz-Read-Tiefe der Mitochondrien und zum Berechnen einer durchschnittlichen Sequenz-Read-Tiefe des Autosoms, und zum Extrahieren der Kopienzahlvarianten der Mitochondrien durch Dividieren der Sequenzinformationstiefe der Mitochondrien durch die durchschnittliche Sequenzinformationstiefe des Autosoms; und
eine Steuereinheit zum Eingeben von mindestens einem der Fragmentlängen und der Kopienzahlvarianten des Geschlechtschromosoms, der Fragmentlängen des P-Arms, der Fragmentlängen des Q-Arms und der Kopienzahlvarianten der Mitochondrien als einen Eingabewert eines vorab erlernten Algorithmus, und zum Ausgeben des Auftretens von Krebs als einen Ausgabewert,
wobei die Steuereinheit so konfiguriert ist, dass sie mindestens eines der Fragmentlängen und der Kopienzahlvarianten des Geschlechtschromosoms, der Fragmentlängen des P-Arms, der Fragmentlängen des Q-Arms, und der Kopienzahlvarianten der Mitochondrien als einen Eingabewert eines künstliche Intelligenz-Algorithmus eingibt und ein Stadium und einen Ursprung von Krebs als einen Ausgabewert ausgibt.

5. Vorrichtung nach Anspruch 4, wobei die DNA-Analyseeinheit ferner so konfiguriert ist, dass sie:
die Sequenzinformationen unter Verwendung von menschlichen Referenzgenomdaten ausrichtet; und
die Fragmentlängen des Autosoms und des Geschlechtschromosoms von einer ersten Länge zu einer zweiten Länge unter Verwendung der ausgerichteten Sequenzinformationen extrahiert.

6. Vorrichtung nach Anspruch 4, wobei der vorab erlernte Algorithmus und der künstliche Intelligenz-Algorithmus jeweils mindestens einen Algorithmus von Random Forest (RF), Support Vector Machine (SVM), eXtra Gradient Boost (XGB), Decision Tree Classifier (DTC), K-nearest Neighbors (KNN), Gaussian Naive Bayes (GNB), Stochastic Gradient Descent (SGD), Linear Discriminant Analysis (LDA), Ridge Regression, Lasso und Elastic net einschließen.

## Revendications

1. Un procédé de diagnostic du cancer en utilisant des données de biopsie liquide, mis en oeuvre par un dispositif, le procédé comprenant :
a) le fait d'acquérir des informations de séquence d' ADNct (ADN tumoral circulant) à partir de plasma extrait du sang, et d'extraire des longueurs de fragments et des variations du nombre de copies d'un chromosome sexuel, d'un autosome et de mitochondries sur la base des informations de séquence acquises ; et
b) le fait d'extraire des lectures de fragments et des longueurs de fragments du bras P et du bras Q sur la base d'un centromère du chromosome sexuel
c) le fait de calculer une profondeur de lecture de séquence des mitochondries, et de calculer une profondeur moyenne de lecture de séquence de l'autosome et d'extraire les variations du nombre de copies des mitochondries en divisant la profondeur d'informations de séquence des mitochondries par la profondeur moyenne d'informations de séquence de l'autosome ; et
d) le fait de saisir au moins l'une des longueurs de fragments et des variations du nombre de copies du chromosome sexuel, des longueurs de fragments du bras P, des longueurs de fragments du bras Q et des variations du nombre de copies des mitochondries en tant que valeur d'entrée d'un algorithme pré-appris, et de délivrer en sortie l'occurrence d'un cancer en tant que valeur de sortie ; et
e) le fait d'entrer, en tant que valeurs d'entrée d'un algorithme d'intelligence artificielle, au moins une des longueurs de fragments et des variations du nombre de copies du chromosome sexuel, des longueurs de fragments du bras P, des longueurs de fragments du bras Q et des variations du nombre de copies des mitochondries, et de délivrer en sortie un stade et une origine du cancer en tant que valeur de sortie.

2. Le procédé selon la revendication 1, dans lequel l'opération a) comprend :
le fait d'aligner les informations de séquence en utilisant des données de génome humain de référence, et
le fait d'extraire les longueurs de fragments de l'autosome et du chromosome sexuel d'une première longueur à une deuxième longueur en utilisant les informations de séquence alignées.

3. Le procédé selon la revendication 1, dans lequel dans l'opération d), l'algorithme pré-appris et dans l'opération e), l'algorithme d'intelligence artificielle comprennent au moins un algorithme de forêt aléatoire (RF, « Random Forest »), de machine à vecteurs de support (SVM, « Support Vector Machine »), d'amplification de gradient supplémentaire (XGB, « eXtra Gradient Boost »), de classificateur d'arbre de décision (DTC, « Decision Tree Classifier »), de K-plus proches voisins (KNN, « K-nearest Neighbors »), de Bayes Naive gaussien (GNB, « Gaussian Naive Bayes »), de descente de gradient stochastique (SGD, « Stochastic Gradient Descent »), d'analyse discriminante linéaire (LDA, « Linear Discriminant Analysis »), de régression de crête, de lasso et de réseau élastique.

4. Un appareil de diagnostic du cancer en utilisant des données de biopsie liquide, l'appareil comprenant :
une unité d'analyse d'ADN permettant d'acquérir des informations de séquence d'ADNct (ADN tumoral circulant) à partir de plasma extrait du sang, d'extraire des longueurs de fragments et des variations du nombre de copies d'un chromosome sexuel, d'un autosome et de mitochondries sur la base des informations de séquence acquises, d'extraire des lectures de fragments et des longueurs de fragments du bras P et du bras Q sur la base d'un centromère du chromosome sexuel, de calculer une profondeur de lecture de séquence des mitochondries, et de calculer une profondeur de lecture de séquence moyenne de l'autosome et d'extraire les variations du nombre de copies des mitochondries en divisant la profondeur des informations de séquence des mitochondries par la profondeur moyenne des informations de séquence de l'autosome ; et
une unité de commande pour entrer au moins l'une des longueurs de fragments et des variations du nombre de copies du chromosome sexuel, les longueurs de fragments du bras P, les longueurs de fragments du bras Q et les variations du nombre de copies des mitochondries en tant que valeur d'entrée d'un algorithme pré-appris, et pour délivrer en sortie l'occurrence d'un cancer en tant que valeur de sortie,
l'unité de commande étant configurée pour entrer au moins l'une des longueurs de fragments et des variations du nombre de copies du chromosome sexuel, les longueurs de fragments du bras P, les longueurs de fragments du bras Q et les variations du nombre de copies des mitochondries en tant que valeur d'entrée d'un algorithme d'intelligence artificielle, et sortir un stade et une origine du cancer en tant que valeur de sortie.

5. L'appareil selon la revendication 4, dans lequel l'unité d'analyse d'ADN est en outre configurée pour :
aligner les informations de séquence en utilisant des données de génome humain de référence ; et
extraire les longueurs de fragments de l'autosome et du chromosome sexuel d'une première longueur à une deuxième longueur en utilisant les informations de séquence alignées.

6. L'appareil selon la revendication 4, dans lequel l'algorithme pré-appris et l'algorithme d'intelligence artificielle comprennent chacun au moins un algorithme de forêt aléatoire (RF, « Random Forest »), de machine à vecteurs de support (SVM, « Support Vector Machine »), d'amplification de gradient supplémentaire (XGB, « eXtra Gradient Boost »), de classificateur d'arbre de décision (DTC, « Decision Tree Classifier »), de K-plus proches voisins (KNN, « K-nearest Neighbors »), de Bayes Naive gaussien (GNB, « Gaussian Naive Bayes »), de descente de gradient stochastique (SGD, « Stochastic Gradient Descent »), d'analyse discriminante linéaire (LDA, « Linear Discriminant Analysis »), de régression de crête, de lasso et de réseau élastique.
